# EUROPEAN PATENT APPLICATION

(11) **EP 4 763 159 A2**
(43) Date of publication of application: **24.06.2026**
(21) Application number: 26176650.5
(22) Date of filing: 07.09.2023
(51) Int. Cl.: A61F 9/008

(54) **LASER SYSTEM HAVING A BALANCED SUSPENSION FOR A LASER APPLICATOR AND METHOD OF USING THE LASER SYSTEM**

(30) Priority: 16.09.2022 EP 22196241
(62) Divisional of application: 23767902.2
(71) Applicant: Technolas Perfect Vision GmbH, 80992 München (DE)
(72) Inventor: DONITZKY, Christof, 80992 München (DE); HENNIG, Georg, 80992 München (DE); WÜLLNER, Christian, 80992 München (DE)
(74) Representative: Maiwald GmbH

(57) **Abstract**

The present disclosure relates to an ophthalmic laser system for performing laser treatments of an eye. The laser system includes a base, which houses a laser source of the laser system. The system further includes a laser applicator, which comprises an optical system through which the treatment laser beam exits the laser applicator in a direction towards the patient's eye and a supporting arm. The supporting arm is connected to the laser applicator at a first end of the supporting arm and a second end of the supporting arm is connected to the base. The supporting arm is configured so that the laser applicator is positionable relative to the base in three dimensions. The laser system further comprises a motorized three-axis positioning system, which is operatively coupled to the controller for positioning the laser applicator relative to at least a portion of the supporting arm in three dimensions.

## Description

### Field

The present invention relates to a system for performing laser treatments, such as treatments which form incisions within the cornea or the natural lens of the eye. The present invention also relates to systems for ablating a surface portion of the cornea, such as photorefractive keratectomy (PRK) and laser in situ keratomileusis (LASIK) treatments.

### Background

Cataract surgery is one of the most common ophthalmic surgical procedures performed. The primary goal of cataract surgery is the removal of the aging lens and replacement with an artificial lens or intraocular lens (IOL) that restores some of the optical properties of the aged lens.

The major steps in cataract surgery consist of forming a corneal incision to allow access to the anterior chamber of the eye and incisions to correct for astigmatism (Limbal relaxing incisions, abbreviated as LRIs or astigmatic keratotomy, abbreviated as AK), cutting and opening the capsule of the lens to gain access to the lens (capsulotomy), lens fragmentation and removing the fragmented lens through phacoemulsification, irrigation and aspiration and in most cases placing an artificial intraocular lens in the eye.

Therefore, femtosecond laser-assisted cataract surgery is a multi-stage process which includes femtosecond laser surgery (LRI, AK, corneal incision, capsulotomy and lens fragmentation), a phacoemulsification process (emulsifying, liquifying and aspirating the lens) and manual surgical procedures (implantation of the intraocular lens).

Using conventional femtosecond laser systems, this, however, typically requires that the patient is moved from his regular patient bed to a separate stationary patient support which is part of the femtosecond laser system and which is specifically adapted for conducting the laser surgical steps. Such patient transfer procedures, however, complicate the clinical workflow and are uncomfortable for the patients.

Moreover, in order to reduce footprint and costs, it is desirable that femtosecond laser systems, which are configured for cataract surgery also be used for forming corneal flaps for laser in situ keratomileusis (LASIK). A femtosecond laser for forming LASIK flaps, however, should be located close to the excimer laser system in order to make it easy for the patient to move from one system to the other. On the other hand, a femtosecond laser for cataract surgery should be located in the sterile operating theatre, where phacoemulsification and IOL implantation are carried out.

In view of the above, there is a need to provide a laser system which allows an efficient clinical workflow.

### Summary

Embodiments of the present invention pertain to an ophthalmic laser system for performing laser treatments of an eye. The laser system includes a base, which houses at least a portion of a laser source of the laser system wherein the laser source is configured for generating a treatment laser beam for performing the laser treatments. The laser system further includes a laser applicator, which comprises an optical system through which the treatment laser beam exits the laser applicator in a direction toward the patient's eye. The laser system further includes a supporting arm and a controller. The supporting arm is connected to the laser applicator at a first end of the supporting arm. A second end of the supporting arm may be connected to the base, and, additionally or alternatively, the supporting arm comprises an interface for connecting the supporting arm to a further component at a second end of the supporting arm. The supporting arm may be configured so that the laser applicator is positionable relative to the base in three dimensions. The laser applicator may be positionable while maintaining a vertical orientation of the laser applicator. The laser system may further comprise a motorized three-axis positioning system, which is operatively coupled to the controller for positioning the laser applicator relative to at least a portion of the supporting arm or relative to the entire supporting arm. The positioning system may be configured so that the laser applicator is positionable in three dimensions.

The laser system may include an articulated beam guide tube. At least a portion of the articulated beam guide tube may extend between a first location where the treatment laser beam exits from the base or from the supporting arm and a second location, where the treatment laser beam enters into the supporting arm or into the laser applicator. The laser system may include a coupling arrangement for coupling the articulated beam guiding tube to the supporting arm at one or more locations along the beam guiding tube between the first location and the second location.

The supporting arm may be connected to the laser applicator at a first end. The second end of the supporting arm may be connected to the base and/or may be connectable to a further component at the second end of the supporting arm. The further component may be external to the ophthalmic laser system. By way of example, the further component may be a second base or may be a wall, a ceiling or a floor of a building.

The base of the laser system may be configured as a moveable base, which is moveable across the floor surface. The mobile base may include a plurality wheels, such as four wheels for moving the base across the floor surface. The mobile base may be configured so that the entire laser surgical system can be moved by one person across the floor. The supporting arm and the laser applicator may only be supported by the base.

The laser source may be an infrared laser source. The infrared laser source may be configured as a femtosecond laser source. A pulse energy of the laser pulses may be greater than 1 nanojoule, or greater than 10 nanojoule or greater than 50 nanojoule. The pulse energy may be less than 20 microjoule, or less than 15 microjoule or less than 10 microjoule. A pulse duration of the pulsed laser beam may be less than 800 femtoseconds, or less than 500 femtoseconds, or less than 300 femtoseconds, or less than 150 femtoseconds, or less than 100 femtoseconds. The pulse duration may be greater than 10 femtoseconds or greater than 50 femtoseconds. A repetition rate of the pulsed laser beam may be greater than 50 kHz or greater than 80 kHz. The repetition rate of the pulsed laser beam may be less than 10 MHz or less than 1 MHz. A center wavelength of the pulsed laser beam, which is incident on the eye may be in a range of between 800 nanometers and 1400 nanometers, or between, 900 nanometers and 1400 nanometers, or between 1000 nanometers and 1100 nanometers, or between 1010 nanometers and 1050 nanometers.

The infrared laser source may be configured so that the laser pulses have a pulse energy so that the laser beam generates photodisruption within corneal tissue or within the natural lens of the patient's eye. The photodisruption may be caused by laser-induced optical breakdown. Alternatively, a pulse energy of the laser pulses may be below a threshold for generating laser-induced optical breakdown. By way of example, a plurality of pulses, which have a pulse energy below the threshold for generating laser-induced optical breakdown may be overlapped in a manner so as to generate tissue separation within the cornea.

The infrared laser source may include a precompensator for at least partially pre-compensating a change of the group delay dispersion (GDD) of the laser pulses, which is induced by components of the laser optical system, which are in the beam path of the laser beam downstream of the laser source. If a laser pulse has a positive GDD, longer wavelengths of the laser pulse propagate faster than shorter wavelengths. A positive group delay dispersion therefore corresponds to a material dispersion, which is typical in transparent media, since red wavelengths experience a lower refractive index compared to blue wavelengths. The pre-compensator may be configured to reduce the group delay dispersion. By way of example, the reduced group delay dispersion generated by the pre-compensator may have a lower positive or a more negative group delay dispersion.

A lateral diameter of the focus of the treatment laser beam of the infrared laser source within the cornea or the lens may be smaller than 10 micrometers, or smaller than 6 micrometers. The diameter may be greater than 3 micrometers. The lateral diameter may be measured in a direction perpendicular to an optical axis of the laser optical system. The lateral diameter may be measured as an 80% encircled energy diameter.

Alternatively, the laser source may be configured as an excimer laser source. The laser system may be configured to generate a laser beam, which is focused on the anterior surface of the eye. A wavelength of the treatment laser beam generated by the excimer laser source may be greater than 150 nm or greater than 190 nm. The wavelength may be less than 400 nm or less than 200 nm. A pulse duration of the treatment laser beam generated by the excimer laser source may be shorter than 100 ns or shorter than 50 ns. The pulse duration may be greater than 1 ns or greater than 3 ns.

The controller may include a data processing system. The data processing system may include a computer system having a processor and a memory for storing instructions processable by the processor. The processor may execute an operating system. The data analysis system may further include a user interface configured to allow a user to receive data from the data processing system and/or to provide data to the data processing system. The user interface may include a graphical user interface.

The controller may be configured to determine a scanning path of the pulsed laser beam for scanning the laser focus on or within the cornea or within the natural lens of the patient's eye. The controller may be configured to determine the scanning path based on patient specific data.

The controller may be configured to generate the scanning pattern so that the laser pulses are overlapping or non-overlapping. A lateral displacement of neighboring laser pulses may be less than 30 micrometers, or less than 20 micrometers, or less than 10 micrometers. The displacement may be greater than 0.5 micrometers or greater than 1 micrometer.

The laser source may include an oscillator laser, which is configured to generate a train of low energy ultrashort pulses. A pulse energy of the low energy ultrashort pulses may be lower than 100 nJ or lower than 20 nJ, or lower than 10 nJ. The pulse energy may be greater than 1 pJ or greater than 100 pJ. The laser source may also include an amplifier, such as a regenerative amplifier or fiber amplifier for amplifying at least a portion of the low energy ultrashort pulses.

The base of the laser system may house a portion of the laser source. By way of example, the oscillator laser may be arranged in the base and the amplifier and/or a precompensator of the laser source, which is configured to reduce a group delay dispersion of the treatment laser beam may be arranged in the laser applicator. Alternatively, the base may house the entire laser source, such as the oscillator laser, the amplifier and the precompensator.

The base may include one or more housings. Each of the housings may house a portion of the laser source. Bay way of example, a first housing of the base may house a seed laser of the laser source and a second housing of the base may house an amplifier of the laser source, in particular a regenerative amplifier or a fiber amplifier.

The supporting arm may include one or more arm segments. The supporting arm may include a plurality of arm segments, which are connected in series. Neighboring arm segments of the supporting arm may be connected via one or more joints. An arm segment may be defined as a member of the supporting arm, which provides a non-articulated connection between a first end of the arm segment and a second end of the arm segment. The arm segment may provide a rigid or telescoping coupling between the first end of the arm segment and the second end of the arm segment. At one or at both ends of the arm segment, the arm segment may be attached or integrally connected to a joint. The term "integrally connected" is meant to indicate that a first element/feature extends or transitions in a continuous manner from a second element/feature and not as two separate and distinguishable elements.

The supporting arm may include at least one arm segment, which forms part of a multi bar linkage of the supporting arm. The multi bar linkage may be configured so that an orientation of a first end of the multi bar linkage relative to a second end of the multi bar linkage is maintained while moving the first end relative to the second end. The multi bar linkage may be a parallel linkage and/or a four bar linkage. The multi bar linkage may be balanced in order to provide at least partial gravity counterbalancing for the laser applicator.

The arm segments may be connected in series via rotational joints. Each of the rotational joints may be configured so that an orientation of neighboring arm segments relative to each other is adjustable. Each of the rotational joint may have either one or two rotation axes. The supporting arm may include at least one multi-axis joint, which has two or more rotation axes. Each of the rotation axes may be oriented substantially orthogonal or orthogonal relative to each other. One of the two rotation axes may be substantially oriented or oriented along a vertical direction.

The supporting arm may be configured so that a distance measured along a horizontal between the laser applicator and a location where the supporting arm is connected to the base may be adjustable. A maximum horizontal distance between the base and the applicator head may be greater than 50 centimeters or greater than 10 centimeters. The distance may be smaller than 5 meters or smaller than 3 meters.

The supporting arm may further be configured so that the laser applicator is movable in a circular or substantially circular arc path of travel. The arc may be in a horizontal or substantially horizontal plane. A radius of the arc may be greater than 10 centimeters or greater than 50 centimeters. The radius may be smaller than 5 meters or smaller than 3 meters. The radius may be defined by a longitudinal extent of an arm segment of the supporting arm, wherein the supporting arm is configured so that the arm segment can be rotated about a horizontal rotation axis and/or about a vertical rotation axis. Alternatively, the supporting arm may be configured so that the arm segment can be rotated about a substantially horizontal rotation axis and/or about a substantially vertical rotation axis. The arm segment may be part of a multi bar linkage, in particular part of a four bar or parallel linkage. Additionally or alternatively, the arm segment may be balanced for providing at least partial gravity counterbalancing for the laser applicator.

The positioning system may include, for each of the three axes, a guide, in particular a linear guide. The axes of the positioning system may be mutually perpendicular to each other. The positioning system may be configured as an XYZ positioning system, wherein the Z-axis is a vertical or substantially vertical axis. For each of the guides, the respective guide may be configured as a sliding guide and/or a roller guide. Each of the guide may include two mating guide members. The first guide member may be configured as a rail, may form a guide track and/or may define a guiding path. The second guide member may be configured as a carriage and/or may be configured to be movable along the guiding path and/or the guide track. The carriage may be a slide carriage and/or a roller carriage.

For each of the three axes of the positioning system, the range of movement may be less than 500 millimeters or less than 150 millimeters. The range of movement may be at least 1 millimeter or at least 3 millimeters.

For each of the three axes of the position mechanism, a positioning accuracy may be worse than 1 micrometer or worse than 10 micrometers. The positioning accuracy may be better than 500 micrometers or better than 100 micrometers.

For one or more or each of the axes, a positioning velocity may be user adjustable in a continuous or stepwise manner. The laser system may be configured to receive user input for adjusting a positioning velocity of one or more or each of the axes of the positioning system.

The positioning system may be arranged between the supporting arm and the laser applicator. However, it is also conceivable that the positioning system is part of the supporting arm.

According to an embodiment, the laser applicator comprises a manually operable control unit which is operatively coupled to the controller for performing the positioning of the laser applicator relative to at least the portion of the supporting arm based on user input received via the control element. The manually operated control unit may be configured for directional control, in particular for directional control in three dimensions. By way of example, the manually operated control unit may include a joystick and/or nay include one or more buttons. Each of the button may correspond to a direction of travel.

According to a further embodiment, the laser applicator includes an imaging system for acquiring a frontal image of at least a portion of the patient's eye at least during part of the positioning of the laser applicator relative to at least a portion of the supporting arm.

The controller of the laser system may include an image processing algorithm for determining, whether at least a portion of the frontal image is in focus. Additionally or alternatively, the image processing algorithm may be configured to determine one or more parameters, which depend on, or which are indicative of, a level of focus of at least a portion of the image. The image processing algorithm may include a segmentation algorithm for segmenting the frontal image. The image processing algorithm may determine one or more parameters, which depend on, or which are indicative of, a level of focus of one or more of the segmented image regions.

The imaging system may include an image sensor. The image sensor may include a two-dimensional ordered or unordered array of pixels. The image sensor may be sensitive to one or more wavelengths within a range of between 380 nanometers and 950 nanometers or within a range of between 380 nanometers and 1400 nanometers. The imaging system may include an imaging optical system for imaging a tissue portion which is arranged in an object plane of the imaging optical system onto the image sensor. At least a portion of the imaging optical system may be provided by a portion of the focusing optical system for focusing the treatment laser beam into the eye of the patient.

According to a further embodiment, the laser applicator comprises a beam combiner for combining an imaging beam path of the imaging system with a beam path of the treatment laser beam.

The beam combiner may be configured to deflect the treatment laser beam in a direction toward or substantially toward the eye. The beam combiner may include a mirror and/or a prism. The beam combiner may be configured as a dichroic beam combiner. The beam combiner may be in the beam path of the laser beam downstream of the focusing optical system, within the focusing optical system or upstream of the focusing optical system.

A distance of an object plane of the imaging system from the laser applicator may be configured, or may be adjustable so that, the distance substantially corresponds to a distance of the laser applicator from the cornea of the patient's eye during the laser treatment.

According to an embodiment, the laser applicator comprises a display device for displaying the frontal image at least during part of the positioning of the laser applicator relative to the supporting arm. The display device may mounted on or may be integrated within a housing of the laser applicator. The display may be visible to a user during operation of the manually operable control element.

According to a further embodiment, the laser applicator comprises an interaction measuring unit for generating an output signal which depends on at least a parameter of a mechanical interaction between the patient's eye and the laser applicator.

The interaction measuring unit may include a plurality of interaction measuring sensors. The sensors may be circumferentially distributed about an optical axis of laser applicator. The sensors may be circumferentially distributed at equal angles about the optical axis of the laser applicator. Additionally or alternatively, the sensors may be arranged at a same or substantially same radial distance from the optical axis of the laser applicator.

The measured parameter of the mechanical interaction may depend on a force or a directional component of a force, or may be a force or a directional component of a force. The force may occurs between the patient's eye and the laser applicator. Additionally or alternatively, the measured mechanical interaction may depend on a temporal change of the force or is the temporal change of the force between the patient's eye and the laser applicator.

The interaction measuring unit may include a plurality of interaction measuring sensors. One or more of the interaction measuring sensors may include a force sensor, a piezoelectric sensor and/or a strain gauge.

The interaction measuring unit may be configured to measure a magnitude of a projection of the force vector onto the optical axis of the laser applicator (i.e. the section of the optical axis at a location where the treatment laser beam exits from the laser applicator towards the patient's eye) and/or a magnitude of a projection of the force vector onto a plane perpendicular to the optical axis.

The strain gauge may be configured to measure a strain in a sensing material caused by a force. The strain may be compressive or tensile. The strain gauge may be configured as a foil gauge, a semiconductor gauge (which uses the effect of piezoresistivity), or a capacitive strain gauge. The piezoelectric sensor may use the piezoelectric effect in a piezoelectric material, such as quartz. The piezoelectric sensor may measure a compressive force, a tensile force and/or a shear force acting on the piezoelectric sensor. It is also conceivable that the force sensor includes an interferometric strain sensor or measures a force acting on a birefringent material. It is also conceivable that the force sensor is a fiber optic force sensor.

The force sensor may be arranged in a force path between the patient's eye and an optical system which focuses the treatment laser beam within the patient's eye.

According to a further embodiment, the laser applicator comprises a display device. The controller may be configured to generate data representative of graphical and/or textual information using the output signal generated by the interaction measuring unit. Additionally or alternatively, the controller may be configured to display the graphical and/or textual information on the display device at least during part of the positioning of the laser applicator relative to at least the portion of the supporting arm.

The graphical and/or textual information, which is generated using output signals of the interaction measuring unit may depend on the magnitude and direction of the force between the laser applicator and the eye, which may be determined using the interaction measuring unit.

Additionally or alternatively, the graphical and/or textual information may depend on a rate at which the magnitude and/or direction of the measured force changes.

According to an embodiment, the coupling arrangement comprises a tensile force transmitting connection. The tensile force transmitting connection may comprise a tension spring for transmitting the tensile force.

According to a further embodiment, the coupling arrangement comprises a guide. The guide may be configured as a lateral guide. The lateral guide may be configured to limit a lateral movement of a coupling member of the coupling arrangment relative to a longitudinal axis of an arm segment of the supporting arm. The coupling member may be rigidly attached or integrally connected to the articulated beam guide tube.

The lateral guide may guide a movement of the coupling member in a direction parallel or substantially parallel to a longitudinal axis of an arm segment of the supporting arm. The guide may be configured to limit, during a movement of the laser applicator, a variation of a vertical orientation of a plane defined by consecutive arm segments of the articulated beam guide tube.

According to a further embodiment, the laser applicator includes an optical coherence tomography (OCT) system which is configured for acquiring a cross-sectional image of at least a portion of the eye.

A center wavelength of the OCT measuring arm may be within a range of between 750 and 1400 nanometers. The optical coherence tomography system may be configured to acquire a cross-sectional image of the cornea and/or at least a portion of the natural lens of the eye. The OCT system may include a scanner. A scanner of the OCT system may be separate from the scanner of the optical system for scanning the treatment laser beam.

According to a further embodiment, the laser applicator comprises a beam combiner for combining a beam path of a measuring arm of the OCT system with a beam path of the treatment laser beam.

The beam combiner for combining the beam path of the treatment laser beam with the beam path of the measuring arm of the OCT system may be configured as a dichroic beam combiner. At least part of the beam combiner for combining the beam path of the measuring arm of the OCT system with the treatment laser beam may be provided by at least a portion of the beam combiner for combining the imaging beam path of the imaging system with the beam path of the treatment laser beam.

According to an embodiment, the supporting am includes an arm segment which is rotatable about a horizontal or substantially horizontal axis. According to a further embodiment, the arm segment is part of a multi bar linkage, in particular a four bar linkage, such as a parallel linkage. The multi bar linkage may be configured so that during rotation of the arm segment about the horizontal axis, the vertical orientation of the laser applicator is maintained.

According to a further embodiment, the supporting arm comprises a first arm segment and a second arm segment which are connected to each other in series via an intermediate joint. The first arm segment may be rotatable about a vertical or substantially vertical axis and the second arm segment is rotatable about a horizontal or substantially horizontal axis.

According to a further embodiment, the intermediate joint system is configured to allow the second arm segment to rotate (a) about the horizontal or substantially horizontal axis and (b) about a vertical or substantially vertical axis. The intermediate joint system may include two joints, which are rigidly attached or integrally connected to each other. A first one of the two joints has a vertically or substantially vertically oriented rotation axis and the second one of the two joints has a horizontally or substantially horizontally oriented rotation axis. The first joint may be attached or integrally connected to the first arm segment and the second joint may may be attached or integrally connected to the second arm segment.

According to a further embodiment, the second arm segment is part of a multi bar linkage, in particular a four bar linkage such as a parallel linkage.

According to a further embodiment, the supporting arm comprises a counterbalancing mechanism to provide at least partial gravity counterbalancing for the applicator head.

According to a further embodiment, the counterbalancing mechanism include one or more springs. Each of the springs may be configured as a gas spring or as a mechanical spring.

According to a further embodiment, the supporting arm comprises a braking and/or locking system for arresting a movement of the second end of the supporting arm relative to the first end of the supporting arm. The braking and/or locking system may include one or more lockable joints and/or brakes for braking a movement of portions of the joints relative to each other. The locking mechanism of the lockable joint may be based on a positive locking of members of the joint, which are otherwise movable relative to each other.

According to a further embodiment, the laser applicator comprises a manually operable control unit for selectively activating and deactivating the braking and/or locking system based on user input received via the control unit.

According to a further embodiment, the laser system comprises an interaction measuring unit configured for generating an output signal which depends on a mechanical interaction between the patient's eye and the laser applicator. The controller may be operatively connected to the interaction measuring unit and the braking and/or locking system. The controller is configured to receive the output signal generated by the interaction measuring unit and to determine based on the received output signal, whether or not to deactivate the braking and/or locking system.

The articulated beam guiding tube may include one or more joints, each of which connecting neighboring longitudinal tube segments. Each of the tube elements may be define a linear laser beam path, which extends along the longitudinal axis of the tube segment. For each of the tube segments, the respective tube segment may either be rigid or maybe extendable along a longitudinal axis of the respective tube element.

Each of the joints of the articulated beam guiding tube may include a mirror system. The mirror system may include one or more mirrors. The mirror system may be configured to deflect the treatment laser beam which is emitted from a first one of the neighboring tube elements which are connected to the joint into the second one of the neighboring tube elements.

According to a further embodiment, the supporting arm or the laser applicator has a rotational joint, which has a vertically extending rotation axis for rotating the laser applicator about a vertical axis and relative to at least a portion of the supporting arm.

According to a further embodiment, the laser system comprises a locking system which is configured for locking the rotational joint having the vertically extending rotation axis.

According to a further embodiment, the laser applicator includes a focusing optical system for focusing the treatment laser beam within the eye and/or an axial scanning system for scanning the laser focus along an axis of the laser beam; and/or a beam deflection scanning system for scanning the laser beam through deflection of the laser beam.

Further embodiments of the present disclosure pertain to a method of positioning a laser applicator of an ophthalmic laser system relative to a patient's eye. The method comprises positioning a laser applicator relative to the patient's eye using a supporting arm. The supporting arm may be connected to the laser applicator at a first end of the supporting arm and. A second end of the supporting arm (a) may be connected to the base and/or (b) may comprise an interface for connecting the supporting arm to a further component at the second end of the supporting arm. The laser system may be configured for generating a treatment laser beam for performing the laser treatments. The supporting arm may configured so that the laser applicator is positionable relative to the base base while maintaining a vertical orientation of the laser applicator. The method may further comprise positioning the laser applicator relative to the supporting arm using a motorized three-axis positioning system.

According to a further embodiment, the method includes acquiring a frontal image of the eye using an imaging system of the laser applicator. The method may also include displaying, during at least part of the positioning of the laser applicator relative to the supporting arm, the frontal image on a display device of the laser system. The displayed frontal image may be a real-time image.

According to a further embodiment, during the part of the positioning, a distance of the focal plane from the laser applicator substantially corresponds to a pre-defined distance of the laser applicator from the patient's eye, in particular from a cornea of the patient's eye.

According to a further embodiment, the method further comprises generating, by an interaction measuring unit, an output signal, which depends on a mechanical interaction between the patient's eye and the laser applicator. The method may further include determining, using a controller of the laser system, textual and/or graphical information based on the output signal. The method may further include displaying, at least during part of the positioning of the laser applicator, the textual and/or graphical information.

A time span during which the frontal image is displayed and a time span during which the textual and/or graphical information based on the output signals of the interaction unit may be identical, overlapping or non-overlapping.

According to a further embodiment, the method includes generating, by an interaction measuring unit, an output signal, which depends on a mechanical interaction between the patient's eye and the laser applicator. The method may also include determining, using a controller of the laser system and based on the output signal, whether or not to deactivate brakes of the supporting arm, which arrest a movement of the second end of the supporting arm relative to the first end of the supporting arm.

### Brief Description of the Drawings

Figure 1 is a perspective view of a laser system according to an exemplary embodiment.
Figure 2 is a second perspective view of the laser system according to the exemplary embodiment, which is shown in Figure 1 wherein the supporting arm is in a resting position.

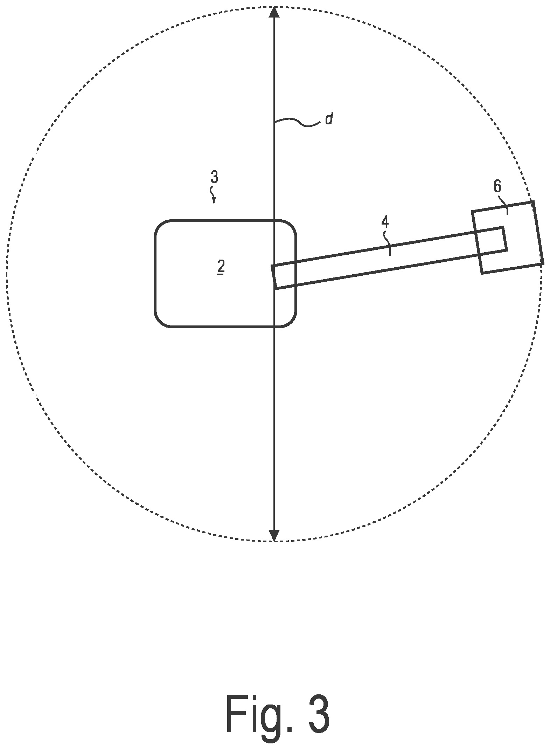

Figure 3 is a schematic view of the laser system of the exemplary embodiment which is shown in Figure 1 and which illustrates the extent of a lateral movement range of the laser applicator of the laser system according to the exemplary embodiment, which is illustrated in Figure 1;
Figure 4 is a further schematic top view of the laser system according to the exemplary embodiment which is shown in Figure 1;
Figure 5 is a schematic side view of the supporting arm and the laser applicator of the laser system according the exemplary embodiment, which is shown in Figure 1;
Figure 6 is a schematic view of information shown on a display device of the laser applicator of the laser system according to the exemplary embodiment, which is shown in Figure 1;
Figure 7 is a schematic view of an imaging system, an OCT system and beam combiners which are arranged in the laser applicator of the laser system according to the exemplary embodiment, which is shown in Figure 1.
Figures 8A and 8B schematically illustrate the interaction measuring unit of the laser applicator of the laser system according to the exemplary embodiment, which is shown in Figure 1;
Figure 9 is a schematic view of information shown on the display device of the laser applicator of the laser system according to the exemplary embodiment, which is shown in Figure 1;
Figure 10 is a schematic view of a parallel linkage and a counterbalancing mechanism of the supporting arm of the laser system according to the exemplary embodiment, which is illustrated in Figure 1; and
Figures 11A to 11C are side views of the supporting arm and an articulated beam guide tube of the laser system and a coupling arrangement for coupling the articulated beam guide tube to the supporting arm of the laser system according to the exemplary embodiment.

### Detailed Description of Exemplary Embodiments

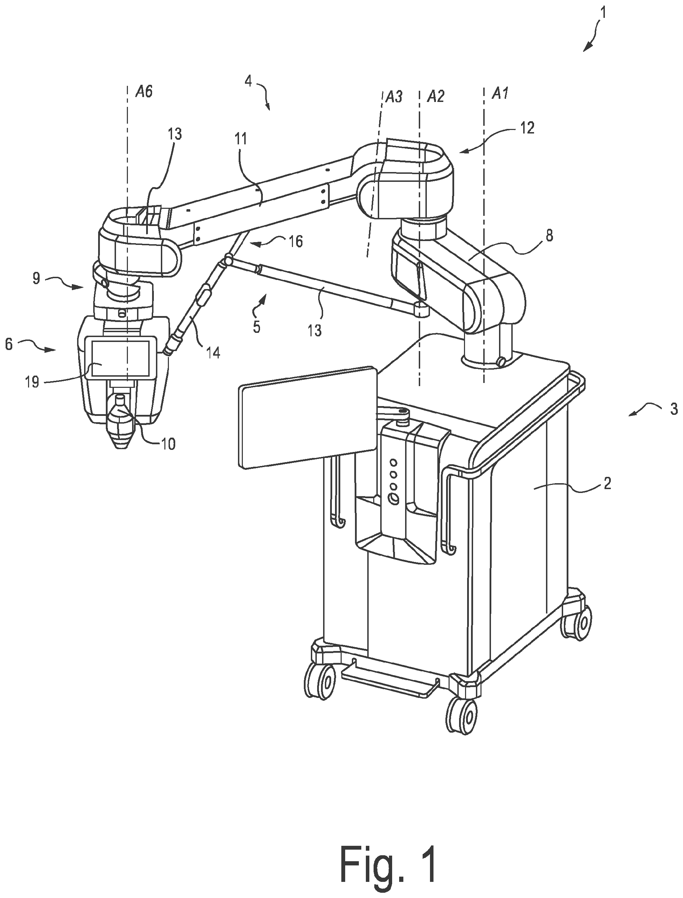

**Figure 1** is a schematic illustration of an ophthalmic laser system 1 according to an exemplary embodiment for performing laser treatments of an eye. The treatments may include, but are not limited to, forming flaps for laser assisted in situ keratomileusis (LASIK) treatments, forming corneal incisions and limbal relaxing incisions (LRI, AK), performing capsulotomy (in particular anterior capsulotomy) and lens fragmentation.

The laser system 1 includes a laser source, which is configured for generating a treatment laser beam for performing the laser treatments. At least a portion of the laser source is mounted within a housing 2 of a base 3. A further portion of the laser source may be arranged within a laser applicator 6, which is supported by an articulated supporting arm 4 and/or within a supporting arm 4, which supports the laser applicator 6. It is also conceivable that the base 3 includes more than one housing, wherein each of them houses a portion of the laser source. By way of example, a first housing of the base 3 houses an oscillator laser of the laser source and a second housing of the base houses an amplifier and/or a precompensator of the laser source.

The laser system 1 comprises a laser optical system which is configured to direct a laser beam towards the eye of a patient who is disposed on a patient bed or support, which is not illustrated in **Figure 1****.** The laser source generates a treatment laser beam, which is guided by the laser optical system through a portion of a supporting arm 4, an articulated beam guide tube 5, and a laser applicator 6. The articulated beam guide tube 5 extends at least between a first location, where the laser beam exits from the supporting arm 4 and a second location, where the laser beam enters into the laser applicator 6. However, the present disclosure is not limited to such a configuration for guiding the treatment laser beam from the base 3 to the patient's eye. By way of example, the laser beam may be guided through the entire supporting arm. In particular, the laser system may be configured so that it does not include a beam guide tube. It is also conceivable that the articulated beam guide tube 5 extends at least between a first location, where the treatment laser beam exits from the base 3 to a second location, where the treatment laser beam enters into the supporting arm 4 or into the laser applicator 6. A portion of the articulated beam guide tube may be arranged within the base 3, within the supporting arm 4 and/or within the laser applicator 6.

In the exemplary embodiment, which is shown in Figure 1, the laser source is configured to emit a pulsed laser beam having a pulse energy and a pulse duration, which is sufficient to generate laser-induced optical breakdown (LIOB) within the cornea or the natural lens of the patient's eye. The laser-induced optical breakdown generated by a laser pulse leads to photodisruption, so that a series of consecutive overlapping or closely located laser pulses generate cuts within the corneal tissue or the natural lens. Photodisruption is a nonthermal process. The laser optical system includes a scanning system, which is configured to scan a focus of the pulsed laser beam within the eye to form perforated cuts or continuous (i.e. non-perforated) cuts. In the exemplary embodiment, which is shown in **Figure 1****,** the scanning system is arranged within the laser applicator 6. However, it is also conceivable that at least a portion of the scanning system is arranged within the base 3 and/or within the supporting arm 4.

It is noted that the present disclosure is not limited to the above laser treatments and laser systems. Specifically, the laser system may be configured to controllably ablate corneal tissue without causing significant damage to adjacent and/or underlying tissues of the eye. The laser system may emit light having a wavelength greater than 150 nm or greater than 190 nm. The wavelength may be less than 400 nm or less than 200 nm. By way of example, the laser source may be configured as an excimer laser source. The laser system may be an argon-fluorine (ArF) excimer laser, which generates pulses of laser light having a wavelength of substantially 193 nm. The laser ablation process may be used for reshaping the cornea. Such ablative treatments may include, but are not limited to, photorefractive keratectomy (PRK), laser assisted subepithelial keratomileusis (LASEK), laser-assisted in-situ keratomileusis (LASIK) and phototherapeutic keratectomy (PTK). In each of these procedures, the laser beam may be used to remove a predetermined amount of the corneal stroma which is located beneath the corneal epithelium and Bowman's membrane to form a reshaped surface portion.

In the exemplary embodiment shown in Figure 1, the laser source is arranged in the base. However it is also conceivable that only a portion of the laser source is arranged in the base and second portion of the source is arranged in the laser applicator 6 or in the supporting arm 4. By way of example, an oscillator laser of the laser source may be arranged in the base and the regenerative amplifier and/or a precompensator of the laser source may be arranged in the laser applicator and/or supporting arm.

Using the supporting arm 4, the laser applicator 6 is positionable in three dimensions. The supporting arm 4 may be configured to allow manual positioning of the laser applicator 6 in three dimensions. However, it is also conceivable that the supporting arm may include one or more motors so that at least a portion of the movements, which can be carried out using the supporting arm 4, are motorized. Between the supporting 4 and the laser applicator 6, a three-axis motorized positioning system 9 is arranged. The positioning system 9 may be configured so that the laser applicator 6 is positionable relative to the supporting arm 4. It is also conceivable that the positioning system 9 is part of the supporting arm 4 so that the laser applicator and a portion of the supporting arm 4 is positionable relative to a further portion of the supporting arm 4. By way of example, the positioning system 9 may be arranged between two arm segments or may be part of an arm segment of the supporting arm 4.

By way of example, as is described in more detail below, the positioning system 9 is used for fine positioning procedure after the laser applicator 6 has been positioned in a course positioning procedure using the supporting arm 4.

The positioning system 9 may be operatively coupled to a controller (not shown in Figure 1). The controller may be arranged within the base 3. However, it is also conceivable that the controller is arranged in a housing which is separate from the base 3 and also separate from the laser applicator 6. The controller may be connected to the base 3, to the positioning system 9 and/or components of the laser applicator 6 via a wired or wireless connection.

The laser applicator 6 may further include a manually operable control unit 10, which is operatively coupled to the controller for performing the positioning of the laser applicator 6 relative to the portion or relative to the entire the supporting arm 4 using the positioning system 9. The manually operable control unit 10 may be configured for directional control. By way of example, the control unit 10 may be configured as a joystick (such as illustrated in **Figure 1****).** However, the present disclosure is not limited to such a control element. It is also conceivable that the control element includes one or more buttons, each of which representing a direction of travel of the laser applicator 6.

The configuration of the ophthalmic laser system according to the exemplary embodiment allows positioning the laser applicator relative to the patient, so that the patient can remain in the regular patient bed where the patient has received pre-surgical treatment. Thereby, patient transfers can be avoided, which otherwise would be required for moving patients from their regular patient beds to a separate stationary patient support which is part of the laser system and specifically provided for conducting the laser surgical steps.

For this reason, the surgical laser system according to the present disclosure not only increases the effectiveness of the clinical workflow but also reduces the footprint required in the surgical operation theatre, since there is no space necessary for a fixedly installed patient support, which is provided solely for performing the laser surgery. Further, a movable laser surgical system facilitates cleaning and sterilization of the surgical operation theatre.

Moreover, in order to reduce footprint and costs, it is desirable that femtosecond laser systems, which are configured for cataract surgery also be used for forming corneal flaps for laser in situ keratomileusis (LASIK). A femtosecond laser for forming LASIK flaps, however, should be located close to the excimer laser system in order to make it easy for the patient to move from one system to the other. On the other hand, a femtosecond laser for cataract surgery, should be located in the sterile operating theatre, where the natural lens is emulsified using a phacoemulsification device and where the intraocular lens is implanted. The laser system according to the exemplary embodiment is a mobile laser system, which can easily be moved between different locations in the hospital. Also, the articulated supporting arm allows the surgeon to more flexibly arrange the laser system within the surgical operating room, where many other devices, such as a surgical microscope, a phacoemulsification system and operating room trolleys are located and where there must be sufficient space for one or more surgeons and other medical staff members.

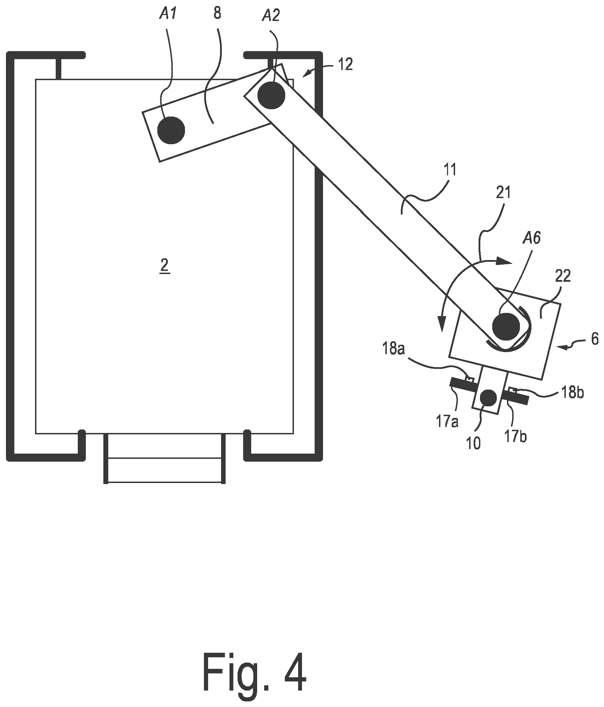

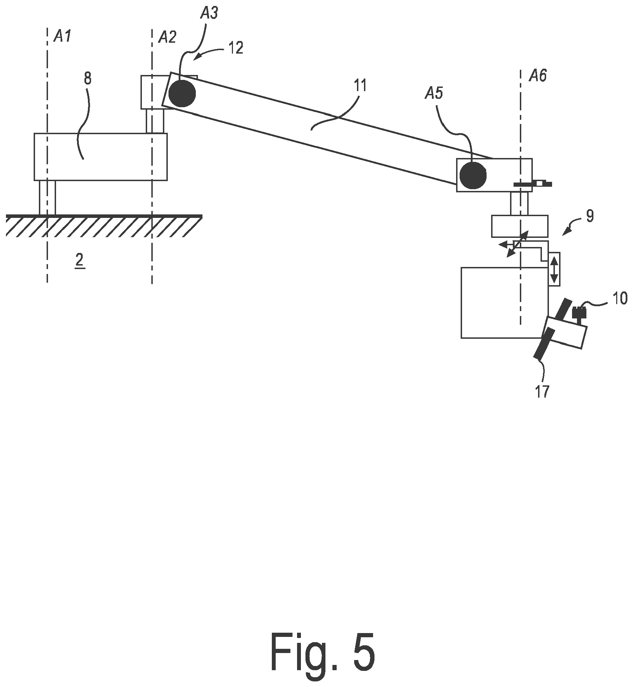

As is illustrated in **Figure 1** and also in the schematic top view of **Figure 4** and the schematic side view of **Figure 5****,** the supporting arm 4 may be configured as an articulated supporting arm having to or more arm segments, which are connected in series. The supporting arm 4 may include a first arm segment 8 and a second arm segment 11. The first arm segment 8 may be rotatably supported by the base 3 so that the first arm segment 8 is rotatable about a first vertical axis *A1.* The first vertical axis *A1* may have a fixed position and orientation relative to the base 3. Additionally or alternatively, the base may be configured so that position, in particular a height and/or orientation (rotation) of the first arm segment 8 relative to the base 3 is adjustable. By way of example, the base 3 may be configured so that the first arm segment is supported by the base using a rotational bearing assembly (not shown in Figure 1) for rotatably supporting the first arm segment 8 about the first vertical axis *A1.* The base 3 may be configured so that a height of the rotational bearing assembly relative to remaining portions of the base 3 is adjustable, e.g. using a motorized heigh adjusting mechanism.

The first arm segment 8 may be connected to the second arm segment 11 via an intermediate joint 12. The intermediate joint 12 may be configured so that an orientation of the second arm segment 11 relative to the first arm segment 8 is adjustable in two dimensions. By way of example, the intermediate joint 12 may be configured so that the second arm segment is rotatable about a vertical axis A2 and also rotatable about a horizontal axis A3.

The supporting arm 4 is configured so that before and after a rotation of the second arm segment 11 about the horizontal axis *A3,* the laser applicator 6 has a same vertical orientation. In the exemplary embodiment, the rotation of the second arm segment 11 about the horizontal axis *A3* is coupled with an orientation of the laser applicator 6 relative to the second arm segment. The coupling is a mechanical coupling which is obtained through a multi bar linkage of the supporting arm 4, which may be configured as a parallel linkage. Two or more parallel bars of the parallel linkage may form the second arm segment 11. However, is is also conceivable that the second arm segment 11 and/or the laser applicator 6 include an inclination sensor for measuring an inclination of the second arm segment 11 and/or the laser applicator 6 relative to the horizontal plane. A controller may be provided which receives output signals from the one or more inclination sensors and which controls an adjustment of the vertical orientation of the laser applicator 6 based on the measured inclination. The adjustment of the vertical orientation may be performed using a motor which drives a rotational joint. It is also conceivable that the connection between the second arm segment 11 and the laser applicator 6 is configured so that the vertical orientation of the laser applicator 6 is maintained by gravitational forces acting on the laser applicator 6.

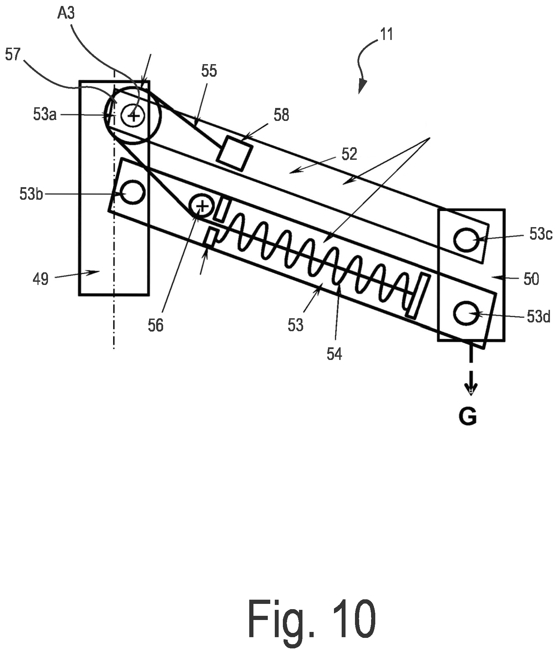

The parallel linkage may be configured so that a distal end of parallel linkage (i.e. distal relative to the base 3) maintains its vertical orientation, irrespective of the orientation of the second arm segment 11 relative to the horizontal plane. An example for a parallel linkage is described below with reference to Figure 10.

As can be seen from **Figures 1****,** **4** **and** **5****,** the laser applicator 6 can be rotated relative to the supporting arm 4 about a vertical rotation axis *A6.* The vertical rotation axis A6 may extend through the laser applicator 6, in particular through a housing 22 of the laser applicator 6. However, it is also conceivable that the vertical rotation axis *A6* does not extend through the laser applicator 6. Rotating the laser applicator about the vertical rotation axis *A6* changes an orientation of the laser applicator 6 relative to the supporting arm 4.

Rotating the laser applicator 6 relative to at least a portion of the supporting arm 4 allows the surgeon during the coarse positioning procedure to adjust the orientation of the laser applicator 6 so that the positioning of the laser applicator 6 relative to the patient's eye is not hindered by space constraints defined by the patient's anatomy. Additionally or alternatively, the laser system may be configured so that the laser applicator 6 can be rotated relative to the supporting arm about a horizontal axis relative to at least a portion of the supporting arm (not shown in Figures 4 and 5). In addition to the laser applicator 6 being rotatable about the horizontal axis, the laser system may also be configured so that the laser applicator 6 is rotatable about a roll axis, which is stationary relative to the laser applicator 6 i.e. stationary for different rotational positions of the laser applicator 6 obtained by the rotation about the vertical and/or horizontal axes (not shown in Figures 4 and 5). This provides an even higher flexibility for adjusting the orientation of the laser applicator 6 relative to the patient.

The laser system according to the exemplary embodiment includes a braking and/or locking system for arresting a movement of the second end of the supporting arm relative to the first end of the supporting arm. Specifically, this allows the surgeon to perform the coarse positioning procedure by manually positioning the laser applicator 6 into a position close to the patient's head. After the course positioning procedure, the surgeon activates a braking and/or locking system of the supporting arm so that in the arrested state, the surgeon can perform the fine positioning procedure using the positioning system 9.

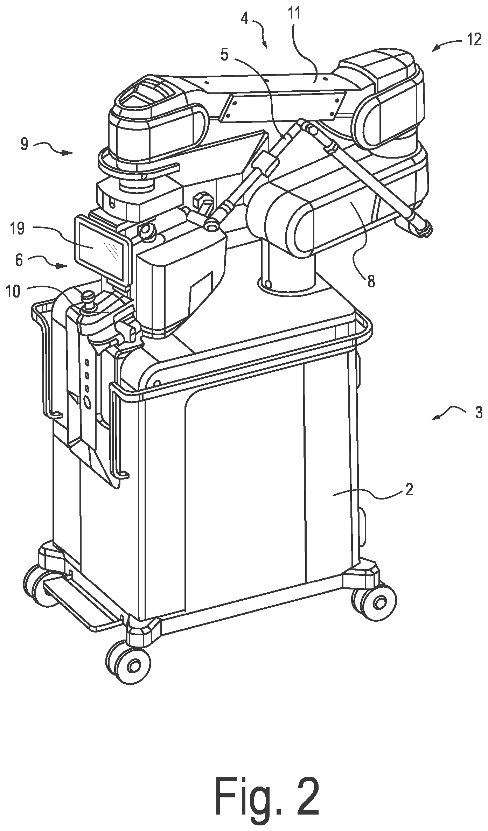

In the exemplary embodiment, the laser applicator 6 includes one or more manually operable control elements 18a, 18b, which allow the surgeon to deactivate the braking and/or locking system so that the applicator head can be positioned in to a different position using the supporting arm 4. In the exemplary embodiment, the laser applicator includes two handles 17a, 17b (shown in Figure 4), which can be grasped by both hands of the surgeon. At each of the handles a release button 18a, 18b is provided. If the surgeon simultaneously presses both release buttons, the braking and/or locking system is deactivated and the surgeon can position the laser applicator 6 in three dimensions. For simplicity of illustration, in Figures 1 and 2, the handles 17a, 17b and the release buttons 18a and 18b are not shown.

The laser system according to the exemplary embodiment is configured to allow positioning the laser applicator relative to the patient's head by performing a coarse positioning procedure in which the laser applicator 6 is adjusted relative to the patient's head using the supporting arm 4. Then, in a subsequent fine positioning procedure using the three-axis positioning system 9, the laser applicator 6 is positioned to its final position relative to the patient's head in which the laser treatment is carried out.

The coarse positioning procedure using the supporting arm 4 allows the surgeon to perform a fast and efficient coarse positioning relative to the patient's head. The manual adjustability also provides increased safety for the patient, since the surgeon can rapidly move the laser applicator 6 to a location distant from the patient's head if needed. However, it is conceivable that one or more joints of the supporting arm are motorized so that the course positioning is performed fully or partially (i.e. using manual positioning) using the motor.

As is explained in more detail further below, the fine positioning procedure may be performed based on images of an imaging system, which is part of the laser applicator 6 and/or based on measurement values of an interaction measurement unit, which measures a mechanical interaction, such as a force, between the patient's eye and the laser applicator 6.

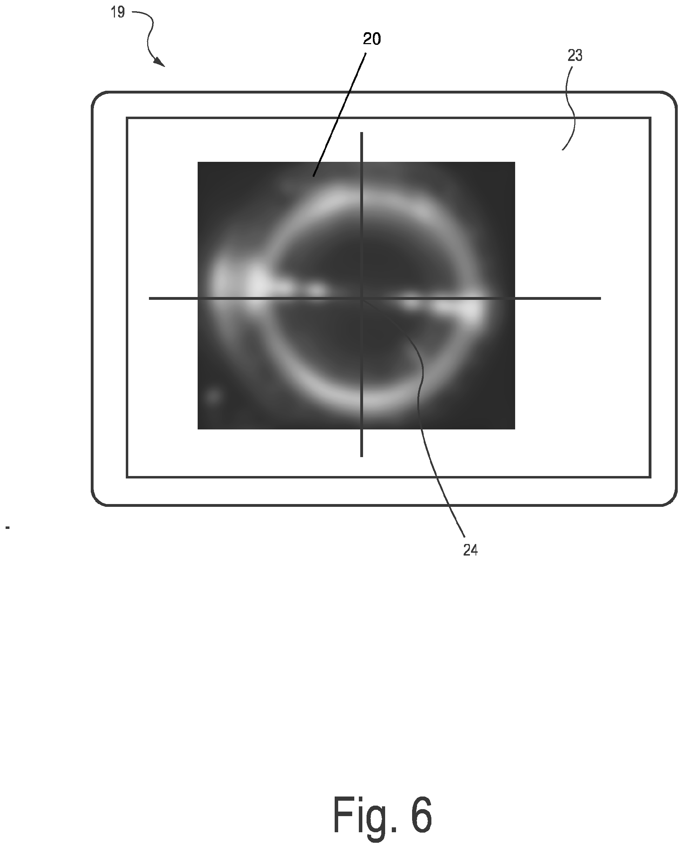

**Figures 6** schematically illustrates information, which is displayed on a display device 19 of the laser applicator 6 during the coarse positioning procedure. As can be seen from Figure 6, the surgeon sees an image on the display device 19, which is generated by an imaging system, which is provided within the laser applicator 6. The imaging system acquires a frontal image 20 of the patient's eye using a constant object plane distance. The object plane distance is adjusted so that it corresponds to a desired pre-defined distance between the laser applicator and the patient's eye. Therefore, using the frontal image of the eye, the surgeon can control a lateral position of the laser applicator using the three-axis positioning system so that the center of the crosshair 24 lies in the center of the pupil of the eye. Further, by adjusting a vertical position of the laser applicator 6 using the positioning system 9 until an in focus image appears on the display device 19, the surgeon can adjust the height of the laser applicator so that the distance between the laser applicator 6 and the patient's eye corresponds to the desired pre-defined distance.

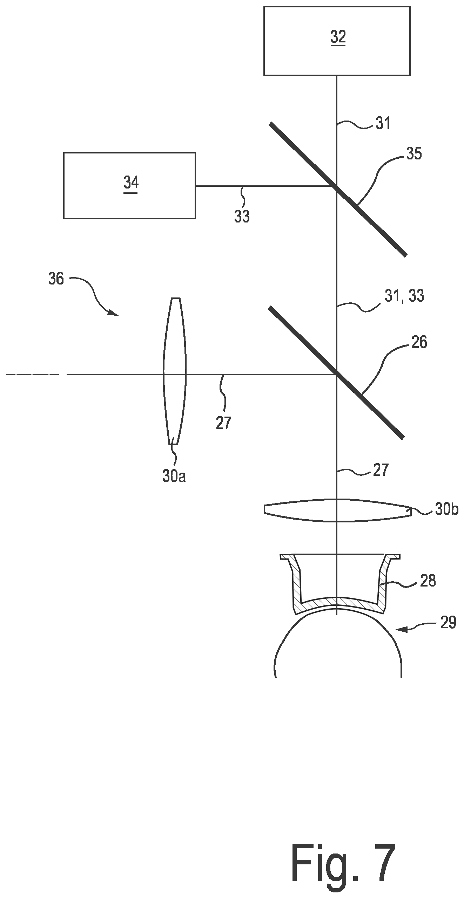

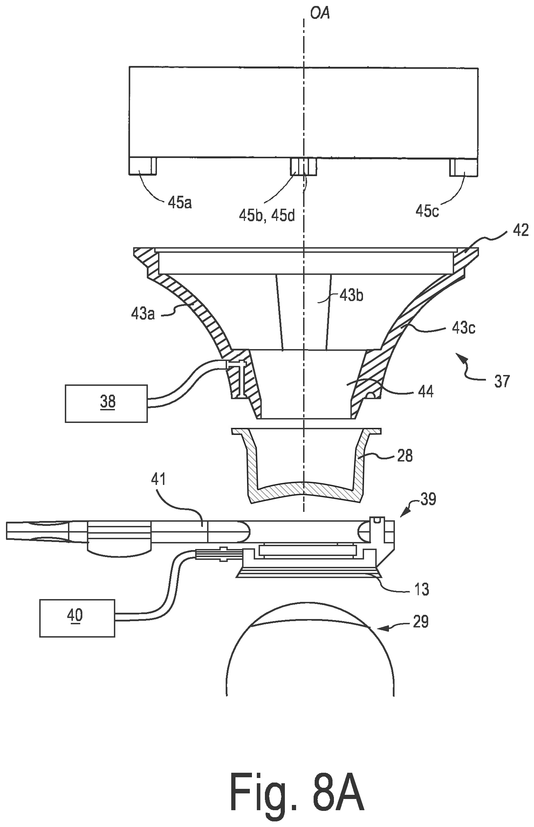

The above course positioning procedure may be carried out in a state in which the suction ring and the contact element (described below with reference to Figures 7 and 8A) are attached to the eye. The laser applicator 6 and the contact element may be configured so that the iris and the limbus are displayed in the frontal image 20. By way of example, the surgeon determines based on a portion or all of these features, whether or not the frontal image 20 is in focus.

The controller of the laser system may include an image processing algorithm for determining, whether at least a portion of the frontal image 20 is in focus and/or which is configured to determine one or more parameters, which depend on, or which are indicative of, a level of focus of at least a portion of the frontal image 20. By way of example, the portion of the frontal image 20 may be the iris of the eye. The image processing algorithm may include a segmentation algorithm for segmenting the frontal image. The image processing algorithm may determine one or more parameters, which depend on, or which are indicative of, a level of focus of one or more of the segmented image regions. By way of example, a segmented image region may represent the iris of the patient's eye.

The controller may be configured to display on the display device 19 graphical and/or textual information based on the determined parameters.

The arrangement of the imaging system within the laser applicator 6 is described in the following with reference to **Figure 7****.** The laser applicator 6 includes a beam combiner 26. The beam combiner 26 is in the beam path of the treatment laser beam 27 between a scanning system (not shown in Figure 7) and a contact element 28 of the patient interface. The contact element 28 includes a concave contact surface, which contacts the anterior surface of the cornea during the treatment. It is noted that the concave shape of the contact element 28, which is shown in Figure 7 is only an example and it is conceivable that the contact surface of the contact element 28 is planar or convex toward the eye.

The beam combiner 26 may be in the beam path of the treatment laser beam 27 between two components 30a and 30b of the focusing optical system 36, as it is illustrated in **Figure 7****.** The focusing optical system 36 is also arranged within the laser applicator 6. It is also conceivable that the laser applicator includes at least a portion of a scanning system for three-dimensionally scan a focus of the treatment laser beam within the eye. The scanning system may include an axial scanning system for scanning the laser focus along an axis of the laser beam and/or a beam deflection scanning system for scanning the laser beam through deflection of the laser beam.

Each of the components 30a and 30b may include one or more optical elements, such as lenses. However, the present disclosure is not limited to such a configuration. It is also conceivable that the beam combiner 26 is either in the beam path of the treatment laser beam 27 between the scanning system and the focusing optical system or in the beam path of the treatment laser beam 27 between the focusing optical system and the contact element 28.

The beam combiner 26 may include a semi-transparent mirror and/or a prism. The semi-transparent mirror may be a dichroic mirror and/or the prism may be dichroic prism. As it is schematically illustrated in **Figure 7****,** the beam combiner 26 may be configured to combine the beam path of the laser beam 27 on the one hand with a measurement beam path 31 of an optical coherence tomography (OCT) system 32 and an imaging beam path 33 of the imaging system 34 on the other hand. The imaging system may have a two-dimensional light sensitive imaging sensor. The light sensitive image sensor may have a two-dimensional array of light sensitive pixels. The optical coherence imaging system may be configured to acquire cross-sectional images of the cornea and/or the natural lens of the eye. The imaging system, which has the imaging sensor may be configured to acquire the two-dimensional frontal image of the eye.

In the eye treatment system according to the exemplary embodiment, the measurement beam path 31 of the optical coherence system 32 and the imaging beam path 33 of the imaging system 34 are combined using a second beam combiner 35, which is outside the beam path of the treatment laser beam 27. The second beam combiner 35 may include a mirror and/or a prism. The mirror may be a dichroic mirror and/or the prism may be a dichroic prism.

The cross-sectional images of the OCT system 32 can be used during the fine positioning procedure for observing, whether or not the anterior surface of the cornea has contacted the contact element 28.

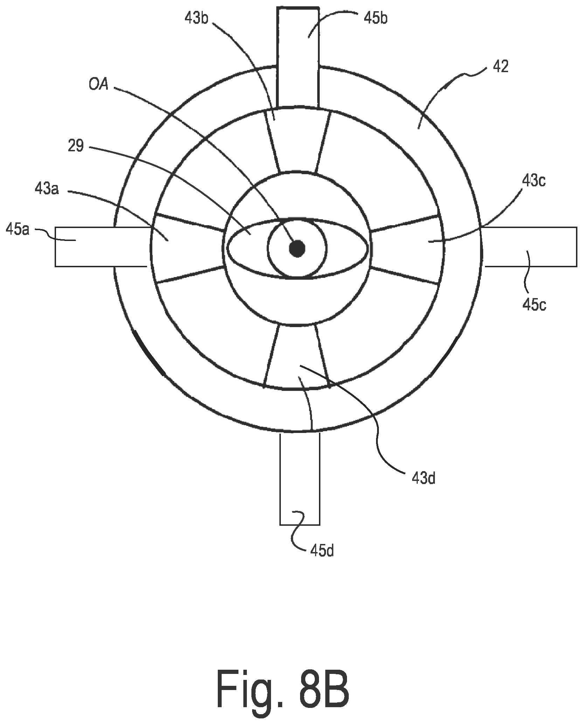

As is discussed in the following with reference to **Figure 8****,** during the fine positioning procedure, the position of the laser applicator relative to the patient's eye is monitored based on signals of an interaction measuring unit, which generates an output signal, which depends on a mechanical interaction between the patient's eye and the laser applicator.

**Figure 8A** schematically illustrates - in an exploded view - the contact element 28 and further components, which are used for coupling the contact element 28 relative to the laser optical system on the one hand and relative to the patient's eye 29 on the other hand. The laser system includes a coupling portion 37, which may be in rigid connection with the laser optical system or which may be displaceably supported in a direction parallel to the optical axis of the laser optical system. The contact element 28 and the coupling portion 37 are configured so that the contact element 28 is detachably coupleable to the coupling portion 37. In the coupled state, the contact element 28 may be in a substantially predefined position relative to the laser optical system and may have a pre-defined inclination relative to the optical axis *OA* of the laser optical system. Alternatively, in the embodiment, in which the contact element 28 is displaceably supported in a direction parallel to the optical axis of the laser optical system, in the coupled state, the contact element 28 is in a pre-defined radial position relative to the optical axis and has a pre-defined inclination relative to the optical axis. The contact element 28 is attached to the coupling portion 37 using a suction mechanism, which includes a suction source 38.

The laser system further includes a suction ring 39 which can be secured to the eye 29 and to which the contact element 28 is rigidly attachable. The suction ring 39 includes a skirt that forms a groove, which defines a suction channel between the skirt and an anterior surface of the eye 29. Generation of a vacuum in the vacuum passage using a vacuum source 40 therefore fixedly attaches the the suction ring 39 to the anterior surface of the eye 29.

The suction ring 39 is rigidly attached to a clamp mechanism 41 or formed with the clamp mechanism 41 as a single piece. The clamp mechanism 41 is used for securing the contact element 28 to the suction ring 39. One example for such a clamp mechanism 41 is disclosed in document US 2007/0093795 A1, the contents of which is incorporated herein by reference for all purposes. However, the present invention is not limited to configurations in which the contact element 28 is secured to the suction ring 39 using a clamp mechanism. Specifically, it is conceivable that the contact element 28 and the suction ring 39 are integrally formed, such as formed as a single piece or integrated into a one-piece assembly.

As can be seen from **Figures 8A** and also from **Figure 8B****,** which is a top view of the coupling portion 37 and the eye 29, the coupling portion 37 includes a coupling ring 42 for coupling the coupling portion 37 to the remaining portion of the laser applicator. Further, the coupling portion 37 includes a plurality of extension arms, 43a, 43b, 43c, 43d, each of which connecting the coupling ring 42 to a lower portion 44 of the coupling portion 37.

When the coupling portion 37 mounted to the remaining portion of the laser applicator, the coupling portion 37 contacts a plurality of force sensors 45a, 45b, 45c, 45d. The force sensors 45a, 45b, 45c, 45d are arranged in a plane which is perpendicular to the optical axis *OA* of the laser applicator and therefore parallel to the plane of the mounting ring 42. As can be seen from **Figure 8B****,** the force sensors are circumferentially distributed at equal angles about the optical axis *OA* of the laser applicator and also at a same distance from the optical axis *OA.* By way of example, each of the force sensors 45a, 45b, 45c and 45d include piezoelectric force sensor, which measures a tensile and/or compressive fore acting on the piezoelectric force sensor. Additionally or alternatively, strain gauges are arranged on one or more of the extension arms.

As can be seen from the cross-sectional illustration of **Figure 8A****,** each of the force sensors 45a, 45b, 45c and 45d is arranged in a force path between the patient's eye 29 and the focusing optical system 36 (shown in Figure 7), which focuses the treatment laser beam within the patient's eye 29 and which is part of the laser applicator. Therefore, a force magnitude, which is measured by each of the respective force sensor, as well as differences between force magnitudes measured by different force sensors can be used for determining the magnitude and direction of the force between the laser applicator and the eye.

Therefore, monitoring the output signals of the force sensors 45a, 45b, 45c and 45d allows docking the laser system to the eye with circumferentially uniform forces which ensure that the eye is not tilted during the laser treatment. Uniform forces measured by the force sensors are particularly important in capsulotomy and lens fragmentation procedures, where a "soft docking" technique is used.

For performing the "soft docking" technique, contact element 28 (shown in Figure 8A) is used, which has a concave contact surface for contacting the anterior surface of the cornea. The forces between the patient's eye and the laser applicator are kept at a low level so that in the docked state, a thin layer of saline solution is present between the contact surface of the contact element and the anterior surface of the cornea of the eye.

Using the output signals of the force sensors for performing the "soft docking" techniques ensures that the vertical force component does not exceed a pre-defined level so that there are only minimal corneal distortions and posterior corneal folds are avoided. Posterior corneal folds can deflect the treatment laser beam which can result in "postage stamp-like" incisions. Further, by ensuring that a lateral component of the force does not exceed a pre-defined level, it is ensured that the eye is not tilted, which increases the accuracy of the laser treatment.

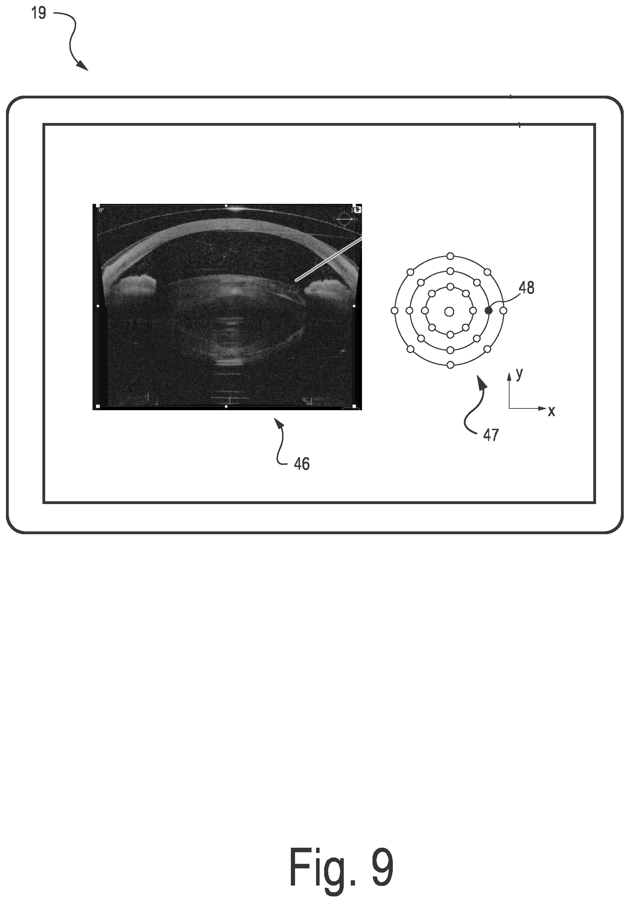

**Figure 9** is a schematic illustration of the information, which is displayed on the display device 19 during the fine positioning procedure. The display device shows an OCT image 46, which is acquired during the docking procedure, as well as a diagram 47, which is generated based on the output signals of the force sensors 45a, 45b, 45c and 45d (shown in Figures 8A and 8B). The OCT image may be a real-time OCT image.

In the exemplary embodiment, the diagram 47 has three concentric rings, wherein an indicator is arranged in the center and each of the rings has eight indicators. After processing of the output signals of the interaction measuring sensors by the controller, one of the indicator is highlighted, such as indicator 48 in Figure 9. The ring on which the highlighted indicator is located indicates the magnitude of the measured force, wherein a greater diameter of the ring indicate a higher force magnitude measured by the force sensors. Specifically, the inner two rings indicate an acceptable force level, wherein the outer ring indicates an unacceptable force level. If the force level is unacceptable, the surgeon uses the positioning system 9 (shown in Figure 1) which is controllable using the control unit 10 to move the laser applicator in a direction away from the patient by raising the laser applicator. Adjusting the laser applicator so that the vertical force level is below a pre-defined level ensures that corneal folds are avoided.

The circumferential position of the highlighted indicator indicates a direction of a lateral component of the force measured using the force sensors. In Figure 9, the highlighted indicator is located on the right side, which indicates to the surgeon that the laser applicator should be moved in the negative x direction in order to minimize lateral forces. If the lateral forces are minimized this avoids that the eye is tilted relative to the optical axis of the laser applicator.

Additionally or alternatively, the controller may be configured to determine a parameter, which is indicative of, or which depends on, a magnitude of at least a component of the force between the laser applicator and the patient's eye. The controller may be configured to display on the display device the parameter. The component of the force may be a component along an optical axis of the laser applicator or a component in a plane, which is oriented perpendicular to the optical axis of the laser applicator.

Therefore, using the force sensors and the three-axis positioning system allows the surgeon to perform a docking procedure which ensures a high quality of the surgical procedure.

In the laser system according to the exemplary embodiment, the output signals of the interaction measuring unit are used to determine, whether to deactivate the braking and/or locking system based on the output signals of the interaction measuring unit. Thereby, it is possible to prevent injuries to the patient's eye in the event that the patient moves his head during the laser treatment. By way of example, if a projection of the force vector onto the optical axis of the laser applicator exceeds a pre-determined threshold, the laser source and the braking and/or locking system is deactivated.

**Figure 10** is a cross-sectional view of the parallel linkage, which includes the second arm segment 11 in the laser system according to the exemplary embodiment, which is shown in Figure 1. The second arm segment 11 comprises two bars 52 and 53 of a four bar linkage, which is configured as a parallel linkage having the bars 52 and 53 of the second arm segment, which are oriented parallel relative to each other and four joints 53a, 53b, 53c and 53d, each of which having a horizontal rotation axis.

The parallel linkage also includes a counterbalancing mechanism for providing a counterbalancing that at least partially counteracts a gravitational force *G* acting on the laser applicator (not shown in Figure 10). In the exemplary embodiment, the counterbalancing mechanism includes a compression spring 54, which is arranged within the first bar 53 and which exerts a pulling force on a toothed belt 55, which extends into the second bar 52 via two toothed belt wheels 56, 57. Within the second bar 52, an end of the toothed belt 55 is fastened using a fastening member 58.

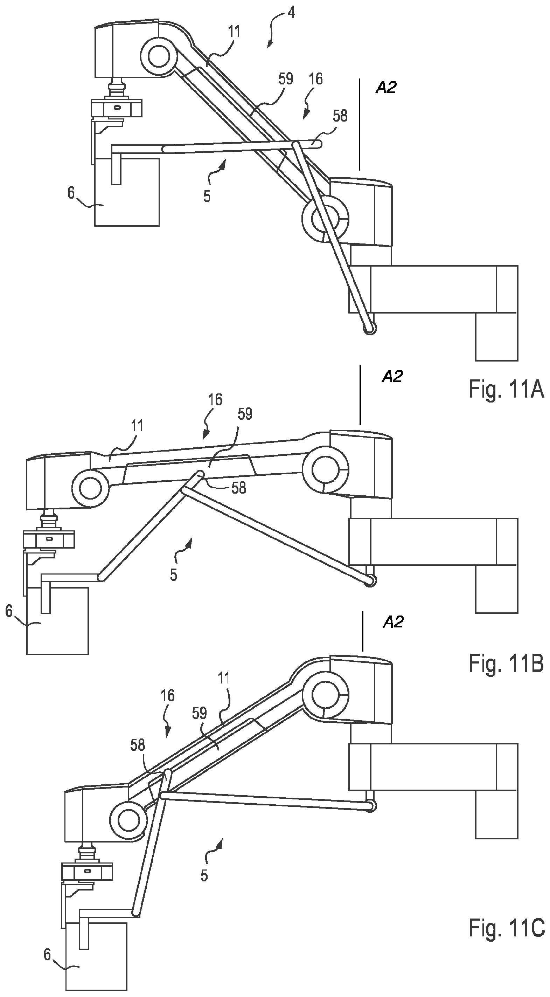

Returning to **Figure 1****,** the laser system according to the exemplary embodiment includes a coupling mechanism for coupling the articulated beam guide tube 5 to the second arm segment 11 of the supporting arm 4. This coupling mechanism 16 is illustrated in more detail in the side views of **Figures 11A to 11C****.**

As can be seen from **Figures 11A to 11C****,** the coupling mechanism 16 includes a first coupling member 58, which is rigidly attached or integrally connected to the articulated beam guide tube 5. Furthermore, the coupling mechanism includes a second coupling member 59, which is a corresponding coupling member to the first coupling member 58 and which is attached or integrally connected to the second arm segment 11. In the exemplary embodiment, the first and second coupling members 58, 59 are configured as a lateral guide wich limits a lateral movement of the first coupling member 59 relative to a direction, which is parallel to a longitudinal axis of the second arm segment 11. In the exemplary embodiment, the coupling mechanism 16 has the effect that a plane, which is defined by the neighboring tube segments 13 and 14 of the articulate beam guide tube 5 has a substantially vertical orientation, for different positions of the laser applicator 6, which are assumed through movements of the first and second arm segments 8 and 11 of the supporting arm 4. This ensures that during operation of the laser system 1, the articulated beam guide tube 5 does not collide with with the supporting arm 4. Such a collision can damage the articulated beam guide tube 5 and/or the supporting arm 4 or can block the positioning of the laser applicator 6 using the supporting arm 4.

Specifically, Figures 11A to 11C schematically illustrate for each of three different configurations of the supporting arm 4 how the fist and second coupling members 58, 59 are positioned relative to each other. For different inclinations of the second arm segment 11, the joint between the first and second arm segment 13 and 14 of the articulated beam guide tube 5 remains coupled to the second arm segment 11 of the supporting arm 1. Further, since the articulated beam guide tube extends between the laser applicator 6 and a location, where the treatment laser beam exits from the first arm segment 8 of the supporting arm 4, the articulated beam guide tube 5 can remain engaged with the linear guide even if the second arm segment is rotated about the vertical axis A2. However, it is noted that if the lateral guide has a sufficient play, the articulated beam guide tube 5 can also be coupled to the second arm segment 11 even if the articulated beam guide tube extends from a location, where the treatment laser beam exits from the base 3.

Additionally or alternatively, it is also conceivable that the coupling arrangement includes a tensile force transmitting connection.

The above embodiments as described are only illustrative, and not intended to limit the technique approaches of the present invention. Although the present invention is described in details referring to the preferable embodiments, those skilled in the art will understand that the technique approaches of the present invention can be modified or equally displaced without departing from the protective scope of the claims of the present invention. In the claims, the word "comprising" does not exclude other elements or steps, and the indefinite article "a" or "an" does not exclude a plurality. Any reference signs in the claims should not be construed as limiting the scope.

Before we go on to set out the claims, we first set out the following clauses describing some prominent features of certain embodiments of the present disclosure.

Clause 1: An ophthalmic laser system for performing laser treatments of an eye, the laser system comprising: a base, which houses at least a portion of a laser source of the laser system wherein the laser source is configured for generating a treatment laser beam for performing the laser treatments; a laser applicator, which comprises an optical system through which the treatment laser beam exits the laser applicator in a direction towards the patient's eye; a supporting arm; and a controller; wherein the supporting arm is connected to the laser applicator at a first end of the supporting arm, and wherein a second end of the supporting arm (a) is connected to the base and/or (b) comprises an interface for connecting the supporting arm to a further component at the second end of the supporting arm; wherein the supporting arm is configured so that the laser applicator is positionable relative to the base in three dimensions while maintaining a vertical orientation of the laser applicator; and wherein the laser system further comprises a motorized three-axis positioning system, which is operatively coupled to the controller for positioning the laser applicator relative to at least a portion of the supporting arm in three dimensions.

Clause 2: The laser system of clause 1, wherein laser applicator comprises a manually operable control unit which is operatively coupled to the controller for performing the positioning of the laser applicator relative to the supporting arm based on user input received via the control element.

Clause 3: The laser system of clauses 1 or 2, wherein the laser applicator comprises an imaging system for acquiring a frontal image of at least a portion of the patient's eye at least during part of the positioning of the laser applicator relative to the supporting arm.

Clause 4: The laser system of clause 3, wherein the laser applicator comprises a display device for displaying the frontal image at least during part of the positioning of the laser applicator relative to the supporting arm.

Clause 5: The laser system of any one of the preceding clauses, wherein the laser applicator comprises an interaction measuring unit for generating an output signal which depends on a parameter of a mechanical interaction between the patient's eye and the laser applicator.

Clause 6: The laser system of clause 5, wherein the output signal depends on a force between the patient's eye and the laser applicator; and/or the interaction determining unit includes a force sensor, a strain gauge sensor and/or a piezoelectric element.

Clause 7: The laser system of clause 5 or 6, wherein the laser applicator comprises a display device, wherein the controller is configured to generate data representative of graphical and/or textual information using the output signal generated by the interaction measuring unit; and to display the graphical and/or textual information on the display device at least during part of the positioning of the laser applicator relative to the supporting arm.

Clause 8: An ophthalmic laser system for performing laser treatments of an eye, the laser system comprising: a base, which houses at least a portion of a laser source of the laser system wherein the laser source is configured for generating a treatment laser beam for performing the laser treatments; a laser applicator, which comprises an optical system through which the treatment laser beam exits the laser applicator towards the patient's eye; a supporting arm, which is connected to the laser applicator at a first end of the supporting arm and wherein the supporting arm (a) is connected to the base and/or (b) comprises an interface for connecting the supporting arm to a further component at a second end of the supporting arm; an articulated beam guide tube, wherein at least a portion of the articulated beam guide tube extends between a first location where the treatment laser beam exits from the base or the supporting arm and a second location, where the treatment laser beam enters into the supporting arm or the laser applicator; wherein laser system includes a coupling arrangement for coupling the articulated beam guiding tube to the supporting arm at one or more locations along the beam guiding tube, between the first location and the second location.

Clause 9: The laser system of clause 8, wherein the coupling arrangement comprises a a tensile force transmitting connection.

Clause 10: The laser system of clause 9, wherein the tensile force transmitting connection comprises a tension spring for transmitting the tensile force.

Clause 11: The laser system of any one of clauses 8 to 10, wherein the coupling arrangement comprises a guide, in particular a lateral guide.

Clause 12: The laser system of clause 11, wherein the guide is configured to limit, during a movement of the laser applicator, a variation of a vertical orientation of a plane defined by consecutive arm segments of the articulated beam guide tube.

Clause 13: The laser system of any one of the preceding claims, wherein the laser applicator comprises an optical coherence tomography (OCT) system which is configured for acquiring a cross-sectional image of at least a portion of the eye.

Clause 14: The laser system of clause 13, wherein the laser applicator comprises a beam combiner for combining a beam path of a measuring arm of the OCT system with a beam path of the treatment laser beam.

Clause 15: The laser system of any one of the preceding clauses, wherein the supporting am comprises an arm segment which is rotatable about a horizontal or substantially horizontal axis.

Clause 16: The laser system of clause 15, wherein the arm segment comprises a parallel linkage mechanism.

Clause 17: The laser system of any one of the preceding clauses, wherein the supporting arm comprises a first arm segment and a second arm segment which are connected to each other in series via an intermediate joint; wherein the first arm segment is rotatable about a vertical or substantially vertical axis and the second arm segment is rotatable about a horizontal or substantially horizontal axis.

Clause 18: The laser system of clause 17, wherein the intermediate joint is configured to allow the second arm segment to rotate about the horizontal or substantially horizontal axis and about a vertical or substantially vertical axis.

Clause 19: The laser system of clause 17 or 18, wherein the second arm segment comprises a parallel linkage.

Clause 20: The laser system of any one of the preceding clauses, wherein the supporting arm comprises a counterbalancing mechanism for providing gravity counterbalancing for the applicator head.

Clause 21: The laser system of clause 20, wherein the supporting arm comprises one or more springs, wherein the supporting arm is configured to provide at least a portion of the gravity counterbalancing using the one or more springs.

Clause 22: The laser system of any one of the preceding clauses, wherein the supporting arm comprises a braking and/or locking system for arresting a movement of the second end of the supporting arm relative to the first end of the supporting arm.

Clause 23: The laser system of clause 22, wherein the laser applicator comprises a manually operable control unit for selectively activating and deactivating the braking and/or locking system based on user input received via the control unit.

Clause 24: The laser system of clause 22 or 23, wherein the laser system comprises an interaction measuring unit configured for generating an output signal which depends on a mechanical interaction between the patient's eye and the laser applicator; wherein the controller is operatively connected to the interaction measuring unit and the brakes; wherein the controller is configured to receive the output signal generated by the interaction measuring unit and to determine based on the received output signal, whether or not to deactivate the braking and/or locking system.

Clause 25: The laser system of any one of the preceding claims, further comprising an articulated beam guide tube, wherein at least a portion of the articulated beam guiding tube extends between a first location on or within the base or an arm segment of the supporting arm and a second location on or within the laser applicator.

Clause 26: The laser system of any one of the preceding claims, wherein the supporting arm has a rotational joint, which has a vertically extending rotation axis for rotating the laser applicator about an axis which extends through the laser applicator.

Clause 27: The laser system of clause 25 or 26, wherein the laser system comprises a locking system which is configured for locking the rotational joint having the rotation axis, which extends through the laser applicator.

Clause 28: The laser system of any one of the preceding clauses, wherein the laser applicator comprises: an objective lens for focusing the treatment laser beam within the eye and/or an axial scanning system for scanning the laser focus along an axis of the laser beam; and/or a beam deflection scanning system for scanning the laser beam through deflection of the laser beam.

Clause 29: A method of positioning a laser applicator of an ophthalmic laser system relative to a patient's eye, the method comprising: positioning a laser applicator relative to the patient's eye using a supporting arm, wherein the supporting arm is connected to a base of the ophthalmic laser system at a first end of the supporting arm; and wherein the supporting arm is connected to the base and/or connectable to a stationary component at a second end of the supporting arm; wherein base houses at least a portion of a laser source of the laser system wherein the laser source is configured for generating a treatment laser beam for performing the laser treatments; wherein the supporting arm is configured so that the laser applicator is positionable relative to the base base while maintaining a vertical orientation of the laser applicator; and positioning the laser applicator relative to at least a portion of the supporting arm using a motorized three-axis positioning system.

Clause 30: The method of clause 29, further comprising: acquiring a frontal image of the eye using an imaging system of the laser applicator; and displaying, during at least part of the positioning of the laser applicator relative to the supporting arm, the frontal image on a display device of the laser system.

Clause 31: The method of clause 30, wherein during the at least the part of the positioning, a distance of the focal plane from the laser applicator substantially corresponds to a pre-defined distance of the laser applicator from the patient's eye.

Clause 32: The method of any one of clause 29 to 31, further comprising: generating, by an interaction measuring unit, an output signal, which depends on a mechanical interaction between the patient's eye and the laser applicator; determining, using a controller of the laser system, textual and/or graphical information based on the output signal; and displaying, at least during part of the positioning of the laser applicator relative to the supporting arm, the textual and/or graphical information.

Clause 33: The method of any one of clauses 29 to 32, further comprising: generating, by an interaction measuring unit, an output signal, which depends on a mechanical interaction between the patient's eye and the laser applicator; determining, using a controller of the laser system and based on the output signal, whether or not to deactivate brakes of the supporting arm, which arrest a movement of the second end of the supporting arm relative to the first end of the supporting arm.

Clause 34: The method of any one of clauses 29 to 33, wherein the output signal depends on a force between the patient's eye and the laser applicator; and/or the interaction determining unit includes a force sensor and/or a strain gauge sensor.

Clause 35: The laser system of any one of clauses 1 to 7, further comprising an articulated beam guide tube, wherein at least a portion of the articulated beam guide tube extends between a first location where the treatment laser beam exits from the base or the supporting arm and a second location, where the treatment laser beam enters into the supporting arm or the laser applicator; wherein laser system includes a coupling arrangement for coupling the articulated beam guiding tube to the supporting arm at one or more locations along the beam guiding tube, between the first location and the second location.

Clause 36: The laser system of clause 35, wherein the coupling arrangement comprises a a tensile force transmitting connection.

Clause 37: The laser system of clause 36, wherein the tensile force transmitting connection comprises a tension spring for transmitting the tensile force.

Clause 38: The laser system of any one of clauses 35 to 37, wherein the coupling arrangement comprises a guide, in particular a lateral guide.

Clause 39: The laser system of clause 38, wherein the guide is configured to limit, during a movement of the laser applicator, a variation of a vertical orientation of a plane defined by consecutive arm segments of the articulated beam guide tube.

## Claims

1. An ophthalmic laser system (1) for performing laser treatments of an eye, the laser system comprising:
a base (3), which houses at least a portion of a laser source of the laser system wherein the laser source is configured for generating a treatment laser beam for performing the laser treatments;
a laser applicator (6), which comprises an optical system through which the treatment laser beam exits the laser applicator (6) towards the patient's eye;
a supporting arm (4), which is connected to the laser applicator (6) at a first end of the supporting arm (4) and wherein the supporting arm (4)
(a) is connected to the base (3) and/or
(b) comprises an interface for connecting the supporting arm (4) to a further component at a second end of the supporting arm (4);
an articulated beam guide tube (5),
wherein at least a portion of the articulated beam guide tube (5) extends between a first location where the treatment laser beam exits from the base (3) or the supporting arm (4) and a second location, where the treatment laser beam enters into the supporting arm (4) or the laser applicator (6);
wherein the laser system (1) includes a coupling arrangement for coupling the articulated beam guiding tube (5) to the supporting arm (4) at one or more locations along the beam guiding tube (5), between the first location and the second location.

2. The laser system (1) of any one of claim 1, wherein the coupling arrangement comprises a guide.

3. The laser system (1) of claim 2, wherein the guide is a lateral guide configured to limit a lateral movement of a coupling member of the coupling arrangement relative to a longitudinal axis of the supporting arm (4).

4. The laser system (1) of claim 2 or 3, wherein the guide is a lateral guide configured to guide a movement of the coupling member in a direction parallel or substantially parallel to a longitudinal axis of an arm segment of the supporting arm (4).

5. The laser system (1) of any one of claims 2 to 4, wherein the guide is configured to limit, during a movement of the laser applicator (6), a variation of a vertical orientation of a plane defined by consecutive arm segments of the articulated beam guide tube (5).

6. The laser system (1) of any one of the preceding claims, wherein the supporting am comprises an arm segment which is rotatable about a horizontal or substantially horizontal axis.

7. The laser system (1) of any one of the preceding claims, wherein the supporting arm (4) comprises a first arm segment and a second arm segment which are connected to each other in series via an intermediate joint;
wherein the coupling mechanism is configured for coupling the articulated beam guide tube (5) to the second arm segment (11).

8. The laser system (1) of claim 7, wherein the coupling arrangement is configured such that for different inclinations of the second arm segment (11), the coupling arrangement remains coupled to the arm segment.

9. The laser system (1) of any one of the preceding claims, wherein the supporting arm (4) comprises a first arm segment (8) and a second arm segment (11) which are connected to each other in series via an intermediate joint (12); wherein the first arm segment (8) is rotatable about a vertical or substantially vertical axis and the second arm segment (11) is rotatable about a horizontal or substantially horizontal axis.

10. The laser system (1) of claim 9, wherein the intermediate joint is configured to allow the second arm segment to rotate about the horizontal or substantially horizontal axis and about a vertical or substantially vertical axis.

11. The laser system (1) of any one of the preceding claims, wherein the supporting arm (4) is connected to the base (3) and wherein the base (3) is configured to be movable across a floor surface.

12. The laser system (1) of any one of the preceding claims, wherein the coupling arrangement comprises a tensile force transmitting connection, wherein preferably, the tensile force transmitting connection comprises a tension spring for transmitting the tensile force.

13. The laser system (1) of any one of the preceding claims, wherein the laser applicator (6) comprises an objective lens for focusing the treatment laser (27) beam within the eye

14. The laser system (1) of any one of the preceding claims, wherein the laser applicator (6) comprises an optical coherence tomography (OCT) system which is configured for acquiring a cross-sectional image of at least a portion of the eye.

15. The laser system (1) of any one of the preceding claims, wherein the laser applicator (6) comprises an axial scanning system for scanning the laser focus along an axis of the laser beam; and/or a beam deflection scanning system for scanning the laser beam (27) through deflection of the laser beam (27).
